(19) Europäisches Patentamt / European Patent Office / Office européen des brevets

(11) **EP 3 822 360 B1**

(12) **FASCICULE DE BREVET EUROPEEN**

(45) Date de publication et mention de la délivrance du brevet:
**14.05.2025 Bulletin 2025/20**

(21) Numéro de dépôt: **20207254.2**

(22) Date de dépôt: **12.11.2020**

(51) Classification Internationale des Brevets (IPC):
**C12Q 1/04** *(2006.01)*    **C12Q 1/18** *(2006.01)*
**C12Q 1/70** *(2006.01)*    **G01N 21/17** *(2006.01)*
**G01N 21/53** *(2006.01)*

(52) Classification Coopérative des Brevets (CPC):
**C12Q 1/04; C12Q 1/18; G01N 21/534;** Y02A 50/30

(54) **PROCÉDÉ DE DÉTERMINATION DE LA SENSIBILITÉ D'UNE SOUCHE BACTÉRIENNE À L'ÉGARD D'UN VIRUS BACTÉRIOPHAGE**

VERFAHREN ZUR BESTIMMUNG DER SENSIBILITÄT EINES BAKTERIENSTAMMS GEGENÜBER EINEM BAKTERIOPHAGEN VIRUS

METHOD FOR DETERMINING THE SENSITIVITY OF A BACTERIAL STRAIN TO A BACTERIOPHAGE VIRUS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **15.11.2019 FR 1912804**

(43) Date de publication de la demande:
**19.05.2021 Bulletin 2021/20**

(73) Titulaires:
- **Commissariat à l'Energie Atomique et aux Energies Alternatives**
  **75015 Paris (FR)**
- **Centre National de la Recherche Scientifique**
  **75016 Paris 16 (FR)**
- **Université Grenoble Alpes**
  **38400 Saint-Martin-d'Hères (FR)**

(72) Inventeurs:
- **PERLEMOINE, Prisca**
  **38054 GRENOBLE cedex 09 (FR)**
- **BORDY, Thomas**
  **38054 GRENOBLE Cedex 09 (FR)**
- **MARCOUX, Pierre**
  **38054 GRENOBLE Cedex 09 (FR)**
- **PICARD, Emmanuel**
  **38054 GRENOBLE cedex 09 (FR)**
- **TOUTAIN, Rémi**
  **38054 GRENOBLE cedex 09 (FR)**
- **ZELSMANN, Marc**
  **38330 Biviers (FR)**

- **MAIRE, Alexis**
  **25300 Granges-Narboz (FR)**

(74) Mandataire: **INNOV-GROUP**
  **209 Avenue Berthelot**
  **69007 Lyon (FR)**

(56) Documents cités:
  **EP-A1- 2 261 379    EP-A1- 2 669 678**
  **EP-A1- 2 747 578    WO-A1-2019/018886**
  **FR-A1- 3 046 239**

- **PRISCA PERLEMOINE: "Optical phage susceptibility testing: developing instrumentation for phage therapy", BACTO-GRE 2019, 17 April 2019 (2019-04-17), XP055715755**
- **MATTHEW HENRY ET AL: "Development of a high throughput assay for indirectly measuring phage growth using the OmniLog TM system", BACTERIOPHAGE, vol. 2, no. 3, July 2012 (2012-07-01), pages 159 - 167, XP055717274, DOI: 10.4161/bact.21440**
- **LUIS A. ESTRELLA ET AL: "Characterization of novel Staphylococcus aureus lytic phage and defining their combinatorial virulence using the OmniLog system", BACTERIOPHAGE, vol. 6, no. 3, 2 July 2016 (2016-07-02), pages e1219440, XP055717278, DOI: 10.1080/ 21597081.2016.1219440**

- PRISCA PERLEMOINE: "Poster P-119 : Phagogramme Optique : L'imagerie sans lentille comme outil de diagnostic pour la phagothérapie", 39E RÉUNION INTERDISCIPLINAIRE DE CHIMIOTHÉRAPIE ANTI-INFECTIEUSE, 16 December 2019 (2019-12-16), pages 69, XP055715790
- PRISCA PERLEMOINE ET AL: "Phage susceptibility testing with lensless imaging technique (Conference Presentation)", PHOTONIC DIAGNOSIS, MONITORING, PREVENTION, AND TREATMENT OF INFECTIONS AND INFLAMMATORY DISEASES 2020, 10 March 2020 (2020-03-10), pages 13, XP055715784, ISBN: 978-1-5106-3210-3, DOI: 10.1117/12.2543785
- ANONYMOUS: "Protocol: Relative Viral Titering with a Resazurin [alamarBlue ] Cell Viability Assay", 1 September 2016 (2016-09-01), pages 1 - 5, XP093188860, Retrieved from the Internet <URL:https://portals.broadinstitute.org/gpp/ public/dir/download?dirpath=protocols/ production& filename=AlamarBlue_virus_titering_sept2016. pdf> [retrieved on 20240722]

# Description

## DOMAINE TECHNIQUE

Le domaine technique de l'invention est la caractérisation de l'aptitude d'une souche bactérienne à être infectée, puis lysée, par un virus bactériophage.

## ART ANTERIEUR

La phagothérapie est l'utilisation de virus bactériophages, usuellement appelés phages, pour traiter les infections d'origine bactérienne. Il s'agit de mettre en œuvre des phages lytiques, capables d'infecter certaines bactéries, puis de les lyser. Il s'agit d'un traitement relativement ancien, découvert et pratiqué avant l'émergence des antibiotiques. Toutefois, dans la plupart des pays, ce traitement a été quelque peu négligé, au profit du recours aux antibiotiques. A l'heure actuelle, la phagothérapie est considérée comme approche thérapeutique prometteuse pour traiter certaines infections bactériennes, particulièrement adaptée lorsque les bactéries sont résistantes aux antibiotiques.

L'efficacité de la phagothérapie a été démontrée pour le traitement d'infections par des bactéries de type *Pseudomonas aeruginosa, Escherichia coli,* ou *Staphilococcus Aureus,* dans des applications de type greffe de peau pour des grands brûlés ou des pathologies liées au diabète.

Les phages mis en œuvre dans des applications de phagothérapie sont des phages lytiques, de telle sorte que lorsqu'une bactérie a été infectée par un phage, cette dernière est lysée. La lyse résulte de la production d'endolysines. Au cours d'une infection, le métabolisme de la bactérie conduit à une réplication du nombre de phages, sous la forme de virions. Ces derniers sont libérés suite à la lyse de la bactérie. Une infection est donc suivie d'une augmentation de la quantité de phages aptes à infecter, puis lyser d'autres bactéries. Il en résulte une diminution, voire un arrêt de la prolifération bactérienne.

Afin de tester la sensibilité d'une bactérie à un phage, des essais, usuellement désignés par le terme "phagogramme", sont pratiqués en laboratoire. Il s'agit de tester la capacité d'une souche bactérienne à être infectée, puis lysée, par un phage.

Les documents WO2019/0188886, EP2747578 décrivent des observations de lyse de bactéries induites par des bactériophages. Les observations sont effectuées par des méthodes optiques, de type fluorescence ou colorimétrie. Il en est de même des publications Henry M. et al. « Development of a high throughput assay for indirectly measuring phage growth using the Omnilog TM system » ou Estrella Luis et al. « Characterization of novel Staphylococcus aureus lytic phage and defining their combinatorial virulence using the Omnilog system ».

Les essais conduits en laboratoire nécessitent de tester différentes souches virales, avec différents ratios de quantités de phages / bactéries. Le nombre de souches virales peut être compris entre 5 et 100, tandis que 5 à 10 ratios de quantités de phages / bactéries sont généralement testés, et cela pour chaque souche virale. Le nombre d'essais à réaliser est donc élevé.

Le document WO2013027146 décrit une observation d'un échantillon selon une modalité de fluorescence. WO2013027146 est basé sur le recours à un agent exogène, en l'occurrence un marqueur fluorescent, pour caractériser le comportement de bactéries en présence de bactériophages.

Les inventeurs proposent une méthode simple, permettant d'analyser la sensibilité d'une souche bactérienne à l'égard d'une souche virale. La méthode permet un suivi du développement de bactéries et une détection précoce de lyses bactériennes par des phages. Elle peut également adresser simultanément plusieurs combinaisons entre des quantités de phages, éventuellement de différentes souches virales, et des quantités de bactéries, éventuellement de différentes souches bactériennes. L'objectif est de réduire le temps passé pour détecter la sensibilité d'une souche bactérienne à l'égard d'une souche virale.

## EXPOSE DE L'INVENTION

Un objet de l'invention est un procédé de détermination de la sensibilité d'une souche bactérienne à l'égard d'une souche virale de bactériophages, selon la revendication 1.

La largeur de la bande spectrale d'émission est de préférence inférieure à 100 nm ou à 50 nm. La distance entre l'échantillon et le capteur d'image est de préférence inférieure à 5 cm ou inférieure à 1 cm.

L'étape d) peut comporter une détermination d'une atténuation de la lumière, émise par la source de lumière, par l'échantillon, de telle sorte que la souche bactérienne est considérée comme :

- peu ou pas sensible à la souche virale des bactériophages lorsque l'atténuation augmente entre deux instants de mesure successifs ;
- ou sensible à la souche virale des bactériophages lorsque l'atténuation n'augmente pas entre deux instants de mesure successifs.

Selon un mode de réalisation :

- lors de l'étape a), les bactéries et les bactériophages sont mélangés dans une solution aqueuse ;
- l'étape c) comporte une acquisition d'au moins deux images, à des instants de mesure successifs ;
- l'étape d) comporte une détermination d'un descripteur de texturation de chaque image acquise, de telle sorte que la souche bactérienne est considérée comme :

- pas ou peu sensible à la souche virale des bactériophages lorsque l'évolution du descripteur de texturation traduit une augmentation d'une diffusion de la lumière par l'échantillon entre deux instants de mesure successifs ;
- ou sensible à la souche virale des bactériophages lorsque l'évolution du descripteur de texturation traduit une diminution ou une stagnation d'une diffusion de la lumière par l'échantillon entre deux instants de mesure successifs.

Le descripteur de texturation peut être déterminé selon une ou plusieurs régions d'intérêt de l'image.

Selon un mode de réalisation :

- lors de l'étape a), les bactéries et les bactériophages sont mélangés dans une gélose ;
- l'étape c) comporte une acquisition d'au moins une image, postérieurement à l'étape a) ;
- l'étape d), comporte une analyse de chaque image acquise, de façon à identifier des régions d'intérêt claires, chaque région d'intérêt claire correspondant à une infection de bactéries par des bactériophages, formant une plage de lyse, chaque région d'intérêt claire indiquant une sensibilité de la souche bactérienne à l'égard de la souche virale des bactériophages.

Le procédé peut alors comporter :

- un comptage du nombre de régions d'intérêt claires sur au moins une image acquise ;
- une estimation d'un titre infectieux des bactériophages dans l'échantillon à partir du nombre de régions d'intérêt claires comptées.

Selon un mode de réalisation :

- lors de l'étape a), les bactéries sont disposées dans une gélose, et les bactériophages sont disposés dans une solution, l'étape a) comportant un dépôt d'au moins une goutte de la solution sur la gélose ;
- l'étape c) comporte une acquisition d'au moins une image, postérieurement à l'étape a) ;
- l'étape d) comporte une analyse de chaque image acquise, de façon à identifier des régions d'intérêt claires, chaque région d'intérêt claire correspondant à une infection de bactéries par des bactériophages, formant une plage de lyse, de telle sorte que l'apparition, de chaque région d'intérêt claire indique une sensibilité de la souche bactérienne à l'égard de la souche virale des bactériophages.

L'étape a) peut comporter un dépôt de plusieurs gouttes, les gouttes étant espacées les unes des autres, deux gouttes différentes comportant respectivement :

- des bactériophages de différentes souches virales ;

- et/ou différentes concentrations de bactériophages d'une même souche virale.

Selon un mode de réalisation :

- lors de l'étape a), les bactériophages sont disposés dans une gélose, et les bactéries sont disposées dans une solution, l'étape a) comportant un dépôt d'une goutte de la solution sur la gélose;
- l'étape c) comporte une acquisition d'au moins une image, postérieurement à l'étape a) ;
- l'étape d) comporte une analyse de chaque image acquise, de façon à identifier:

  - des régions d'intérêt claires, chaque région d'intérêt claire correspondant à une infection de bactéries par des bactériophages, de telle sorte que chaque région d'intérêt claire indique une sensibilité de la souche bactérienne à l'égard de la souche virale des bactériophages;
  - ou des régions d'intérêt sombres, chaque région d'intérêt sombre correspondant à un développement de bactéries en présence de bactériophages, de telle sorte que chaque région d'intérêt sombre indique une insensibilité ou une faible sensibilité de la souche bactérienne d'intérêt à l'égard de la souche virale des bactériophages.

L'étape a) peut comporter un dépôt de plusieurs gouttes, les gouttes étant espacées les unes des autres, deux gouttes différentes comportant respectivement :

- des bactéries de différentes souches bactériennes
- et/ou différentes concentrations de bactéries d'une même souche bactérienne.

Quel que soit le mode de réalisation, l'échantillon peut être divisé en différentes zones spatiales, séparées les unes des autres de telle sorte que:

- au moins deux zones spatiales différentes comportent respectivement une même souche bactérienne et une concentration différente de bactériophages d'une même souche virale;
- et/ou au moins deux zones spatiales différentes comportent respectivement une même souche bactérienne et des bactériophages de différentes souches virales ;
- et/ou au moins deux zones spatiales différentes comportent respectivement des bactériophages d'une même souche virale et différentes souches bactériennes.

Le procédé est alors tel que chaque zone spatiale est associée à une région d'intérêt de chaque image acquise, deux zones spatiales différentes étant associées à deux régions d'intérêt différentes, de façon que l'analyse d'une même image acquise permet d'obtenir une information relative à la sensibilité d'une souche bactérienne à l'égard d'une souche virale de bactériophages, et cela dans différentes zones spatiales.

L'échantillon peut comporter plus de 10, voire plus de 100, zones spatiales différentes, séparées les unes des autres, chaque zone spatiale étant paramétrée trois paramètres correspondant respectivement à la souche bactérienne d'intérêt, à la souche virale des bactériophages et à la concentration des bactériophages, au moins un paramètre de deux zones spatiales différentes étant différent.

L'échantillon peut être réparti dans différentes chambres fluidiques, d'une carte fluidique, chaque chambre fluidique correspondant à une zone spatiale de l'échantillon.

L'échantillon peut être formé suite à un dépôt de gouttes à la surface d'une gélose, en différentes positions, les gouttes étant espacées les unes des autres, de telle sorte qu'une zone spatiale est définie par chaque position de gouttes. La gélose peut comporter les bactéries et les gouttes peuvent comporter des bactériophages. Alternativement, la gélose peut comporter des bactériophages et les gouttes peuvent comporter des bactéries.

Selon un mode de réalisation, le procédé comporte, préalablement à l'étape a), une étape d'enrichissement comportant :

- un mélange de bactériophages de même souche virale, ou de différentes souches virales, dans une solution aqueuse comportant au moins une souche de bactéries ;
- une incubation ;
- une filtration du mélange, de façon à retenir les bactéries et obtenir une solution enrichie en bactériophages ; de telle sorte que lors de l'étape a), la mise en contact des bactériophages avec les bactéries est effectuée en utilisant la solution enrichie en bactériophages.

La distance de défocalisation image et/ou la distance de défocalisation objet est de préférence inférieure à 5 mm, voire à 1 mm, voire inférieure à 500 $\mu$m. Le plan de l'échantillon est un plan s'étendant dans l'échantillon. Il s'agit de préférence de la surface de l'échantillon la plus proche du capteur d'image. Selon ce mode de réalisation, lors de l'étape c), l'image est acquise selon une configuration défocalisée.

L'invention sera mieux comprise à la lecture de l'exposé des exemples de réalisation présentés, dans la suite de la description, en lien avec les figures listées ci-dessous.

## FIGURES

La figure 1 représente un dispositif permettant une mise en œuvre d'un procédé selon l'invention.

La figure 2A schématise un premier mode de réalisation de l'invention.

Les figures 2B et 2C sont des images obtenues en mettant en œuvre le premier mode de réalisation de l'invention.

La figure 2D est une courbe obtenue expérimentalement en mettant en œuvre le premier mode de réalisation. Elle représente une évolution d'une densité optique en fonction du temps.

Les figures 2E et 2F illustrent des exemples de cartes fluidiques pouvant être mises en œuvre.

Les figures 3A et 3B sont des images obtenues en mettant en œuvre une variante du premier mode de réalisation.

La figure 3C montre une courbe obtenue expérimentalement en mettant en œuvre la variante du premier mode de réalisation. Elle représente une évolution d'un indicateur de texture en fonction du temps.

Les figures 4A et 4B schématisent un deuxième mode de réalisation de l'invention.

Les figures 4C, 4D et 4E sont des images obtenues en mettant en œuvre le deuxième mode de réalisation. Elles montrent une formation de plages de lyse en fonction du temps.

Les figures 5A et 5B schématisent un troisième mode de réalisation de l'invention.

Les figures 5C et 5D sont des images obtenues en mettant en œuvre le troisième mode de réalisation.

Les figures 6A et 6B schématisent un quatrième mode de réalisation de l'invention.

La figure 7 montre les principales étapes de mise en œuvre de l'invention.

La figure 8 montre un autre dispositif permettant une mise en œuvre de l'invention.

## EXPOSE DE MODES DE REALISATION PARTICULIERS

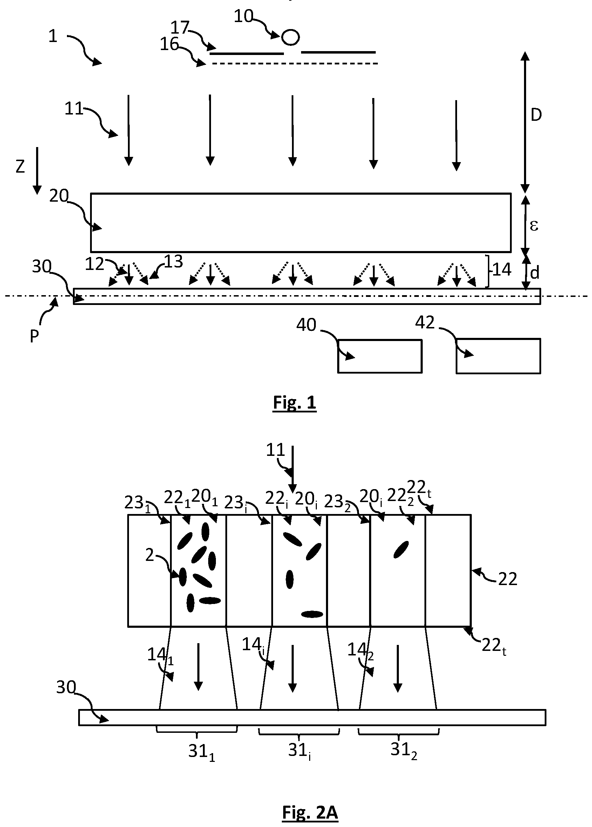

La figure 1 montre un dispositif permettant une mise en œuvre de l'invention. Un échantillon 20 est disposé entre une source de lumière 10 et un capteur d'image 30. La source de lumière 10 produit une onde lumineuse incidente 11, cette dernière se propageant jusqu'à l'échantillon. De préférence, l'onde lumineuse incidente 11 parvient à l'échantillon sous la forme d'une onde plane, ou considérée comme plane.

L'échantillon comporte des bactéries d'une ou plusieurs souches bactériennes, ainsi que des phages (ou bactériophages), d'une ou plusieurs souches virales. L'objectif de l'invention est de visualiser un effet optique, dans l'échantillon, d'une lyse induite par l'infection de bactéries par certains phages. De préférence, les bactéries ne sont pas marquées avec un marqueur colorimétrique ou fluorescent. L'invention est basée sur l'observation des interactions des bactéries avec l'onde lumineuse incidente. Plus précisément, la présence de bactéries dans l'échantillon induit une diffusion de l'onde lumineuse incidente. Plus le nombre de bactéries est élevé, plus l'onde lumineuse incidente est diffusée. Le capteur d'image comporte des pixels, généralement répartis selon un arrangement matriciel dans un plan de détection P. Le capteur d'image collecte onde d'exposition 14 résultant des interactions de l'onde lumineuse incidente 11 dans l'échantillon. L'onde lumineuse d'exposition 14, qui émerge de l'échantillon, est ainsi constituée:

- d'une composante 12 de l'onde lumineuse incidente, non absorbée et non diffusée par l'échantillon. Cette composante est formée de photons usuellement désignés photons balistiques.
- d'une composante 13 résultant de la diffusion de l'onde lumineuse incidente par l'échantillon.

Plus le nombre de bactéries est important, plus la composante de diffusion 13 est importante par rapport à la composante 12 non diffusée.

De préférence, l'onde lumineuse incidente 11 s'étend selon une bande spectrale d'illumination $\Delta\lambda$ s'étendant dans le domaine visible. Elle est de préférence comprise entre 450 nm et 650 nm, et encore de préférence entre 500 nm et 600 nm. C'est en effet entre 500 nm et 600 nm que la diffusion de l'onde lumineuse incidente 11 par les bactéries est maximale. De préférence, la largeur spectrale de la bande spectrale d'illumination est inférieure à 50 nm, voire inférieure à 40 nm. Par largeur spectrale, il est entendu la largeur à mi-hauteur, habituellement désignée par l'acronyme FWHM (Full Width at Half Maximum).

La source de lumière 10 est par exemple une diode électroluminescente ou une diode laser. Il peut également s'agir d'un écran à cristaux liquides, l'avantage étant de disposer d'une surface lumineuse étendue, de quelques cm$^2$ à quelques dizaines de cm$^2$, produisant une illumination uniforme. Un filtre passe-bande 16 peut être interposé entre la source de lumière 10 et l'échantillon 20, de façon à ajuster la bande spectrale de l'onde lumineuse atteignant l'échantillon.

La distance D entre la source de lumière 10 et l'échantillon 20 est ajustée de telle sorte que la source de lumière soit considérée comme ponctuelle. Elle peut être comprise entre 5 cm et 20 ou 30 cm. Un diaphragme 17, définissant une ouverture de diamètre inférieur à 100 $\mu$m ou 200 $\mu$m, peut être interposé entre la source de lumière 10 et l'échantillon 20. De façon alternative, une fibre optique peut être interposée entre la source de lumière et l'échantillon, de façon à former une source de lumière ponctuelle.

Le capteur d'image 30 peut être un capteur de type CCD ou CMOS. De préférence, la surface de détection est supérieure à 10 mm$^2$, voire supérieure à 1 cm$^2$. Les inventeurs ont utilisé un capteur dont la surface de détection est de 14.9 mm x 22.3 mm soit de l'ordre de 3 cm$^2$. Cela permet d'obtenir une image selon un champ d'observation élevée. Afin de maximiser le champ d'observation, la distance d entre l'échantillon 20 et le capteur d'image 30 est aussi faible que possible. Elle est de préférence inférieure à 1 cm. De préférence, l'échantillon 20 est placé au contact du capteur d'image, c'est-à-dire au contact d'une vitre de protection du capteur d'image, ou à quelques millimètres de cette dernière. On remarque l'absence d'optique de grossissement ou de formation d'image entre l'échantillon 20 et le capteur d'image 30. Cela n'empêche pas la présence de microlentilles de focalisation au niveau des pixels du capteur d'image 30. Ainsi, le capteur d'image 30 est disposé selon une configuration d'imagerie sans lentille. Cela permet de maximiser le champ d'observation du capteur d'image. Chaque image permet alors d'adresser un volume important d'échantillon. Le capteur d'image peut être un capteur monochrome ou un capteur couleur. Il permet une formation d'une image représentative de l'onde d'exposition 14.

Le capteur d'image 30 est relié à une unité de traitement 40, recevant les images acquises par le capteur d'image. L'unité de traitement 40 est reliée à une mémoire 42 dans laquelle sont stockées des instructions pour la mise en œuvre de certaines étapes de traitement d'image décrites ci-après.

L'épaisseur $\varepsilon$ de l'échantillon 20 peut varier entre 100 $\mu$m et 1 cm ou 2 cm. La configuration de l'échantillon, et son épaisseur, peut varier selon les modes de réalisation décrits par la suite. D'une façon générale, l'échantillon comporte un support, dans lequel des bactéries, d'au moins une source bactérienne d'intérêt, sont mises en contact avec des phages, d'une souche virale. L'invention est ainsi mise à profit pour déterminer la souche virale et/ou la concentration des phages permettant une lyse des bactéries de la souche bactérienne d'intérêt.

Un premier mode de réalisation est présenté en lien avec les figures 2A à 2F. Selon ce premier mode de réalisation, l'échantillon comporte un mélange de bactéries et de phages mélangés dans une solution, de préférence une solution aqueuse. La solution aqueuse comporte des nutriments, permettant le développement des bactéries. Ainsi, la solution aqueuse comporte un milieu nutritif liquide, de préférence non spécifique, usuellement désigné "bouillon". Il peut par exemple s'agir de trypticase soja, d'un milieu Columbia, ou d'un milieu de type Luria-Bertani.

Selon ce mode de réalisation, l'échantillon est compartimenté selon des chambres fluidiques $22_1$, $22_2$, $22_i$ isolées les unes des autres. L'indice i est un entier désignant le rang d'une carte fluidique, avec $1 \leq i \leq N_i$ où $N_i$ correspond au nombre total de chambres fluidiques. La surface de chaque chambre fluidique, parallèlement au plan de détection P, peut par exemple être comprise entre 1 mm$^2$ et 10 mm$^2$. Les chambres fluidiques sont ménagées dans une carte fluidique 22. De préférence, chaque chambre fluidique est délimitée par une paroi opaque et/ou réfléchissante $23_1$, $23_2$, $23_i$. Cela évite

qu'une lumière, diffusée à l'intérieur d'une chambre fluidique $22_i$, se propage dans une autre chambre fluidique, formant une lumière parasite dans cette dernière. Chaque chambre fluidique $22_i$ comporte un échantillon élémentaire $20_i$ pouvant être différent d'un autre échantillon élémentaire d'une autre chambre fluidique. Pour une souche bactérienne d'intérêt, chaque échantillon élémentaire $20_i$ peut ainsi comporter des phages d'une même souche virale, selon une concentration prédéfinie.

Ainsi, ce mode de réalisation permet de diviser l'échantillon en différentes zones spatiales, chaque zone spatiale correspondant à une chambre fluidique $22_i$, contenant un échantillon élémentaire $20_i$. Chaque échantillon élémentaire peut être paramétré par trois paramètres :

- la souche bactérienne ;
- la souche virale ;
- la concentration des phages de la souche virale.

Entre deux échantillons élémentaires différents, correspondant à deux zones spatiales différentes, au moins un paramètre est différent, sauf dans le cas de répliquas.

Le nombre de chambres fluidiques $22_i$ est de préférence supérieur à 10, et encore de préférence supérieur à 100. Le recours à un capteur d'image de grande surface, tel que précédemment décrit, permet d'acquérir une image adressant simultanément un grand nombre de chambres fluidiques. Cela évite, ou limite, le recours à un mécanisme permettant une translation de l'échantillon 20 relativement au capteur d'image. On comprend que ce mode de réalisation permet d'effectuer un phagogramme. La carte fluidique 22 prend ainsi une forme de plaque à puits, permettant une analyse simultanée d'une multitude d'échantillons élémentaires différents les uns des autres.

La carte fluidique 22 est par exemple réalisée, au moins en partie, dans un matériau plastique transparent, de préférence biocompatible, par exemple COC (Cyclic Olefin Copolymer) ou PMMA (Polymethylmetacrylate). Elle comporte des parois transversales $22_t$ transparentes, qui confinent chaque chambre élémentaire $22_i$. Les parois transversales s'étendent de préférence perpendiculairement à un axe Z selon lequel se propage l'onde lumineuse 11 émise par la source de lumière 10.

Chaque échantillon élémentaire comporte une quantité initiale de bactéries 2. En l'absence de phages, ou en l'absence d'effet notable des phages sur la souche bactérienne, les bactéries prolifèrent. Sur la figure 2A, ce cas correspond à la chambre fluidique $22_1$. Lorsque les phages sont aptes à infecter les bactéries d'intérêt, et à engendrer leur lyse, le nombre de bactéries d'intérêt diminue, ce qui correspond à la chambre fluidique $22_2$. L'augmentation ou la diminution des bactéries entraîne une évolution du transport de la lumière à travers chaque échantillon élémentaire. Les images acquises par le capteur d'image, au cours du temps, permettent d'apprécier et de quantifier cette évolution.

Plus la quantité de bactéries augmente, plus la diffusion de la lumière à travers une chambre fluidique augmente, ce qui réduit l'intensité de l'onde lumineuse d'exposition 14 se propageant vers le capteur d'image 30. Dans l'exemple représenté sur la figure 2A, l'intensité de l'onde lumineuse $14_2$ est supérieure à l'intensité de l'onde lumineuse $14_1$. Le capteur d'image comporte des pixels 31. On peut définir des groupes de pixels $31_i$, chaque pixel d'un même groupe étant exposé à une onde lumineuse d'exposition $14_i$ se propageant depuis une chambre fluidique $22_i$. Ainsi, chaque groupe de pixel $31_i$ est associé à une chambre fluidique $22_i$ et à un échantillon élémentaire $20_i$. En acquérant une image initiale, ou une image d'un échantillon témoin, puis en formant une image au bout d'une certaine durée, il est possible d'évaluer l'évolution de la population bactérienne d'un échantillon élémentaire $20_i$, en déterminant l'évolution temporelle de l'intensité de l'onde lumineuse $14_i$ détectée par les pixels d'un même groupe de pixels $31_i$. A chaque groupe de pixels $31_i$ correspond une région d'intérêt $ROI_i$ de l'image acquise par le capteur d'image 30. Ainsi, l'analyse de chaque région d'intérêt $ROI_i$ de l'image permet de visualiser le développement ou l'inhibition du développement de bactéries dans un échantillon élémentaire $20_i$. Par région d'intérêt, il est entendu des parties de l'image dont l'intensité est considérée comme homogène ou représentative de l'échantillon, ou d'une partie de l'échantillon.

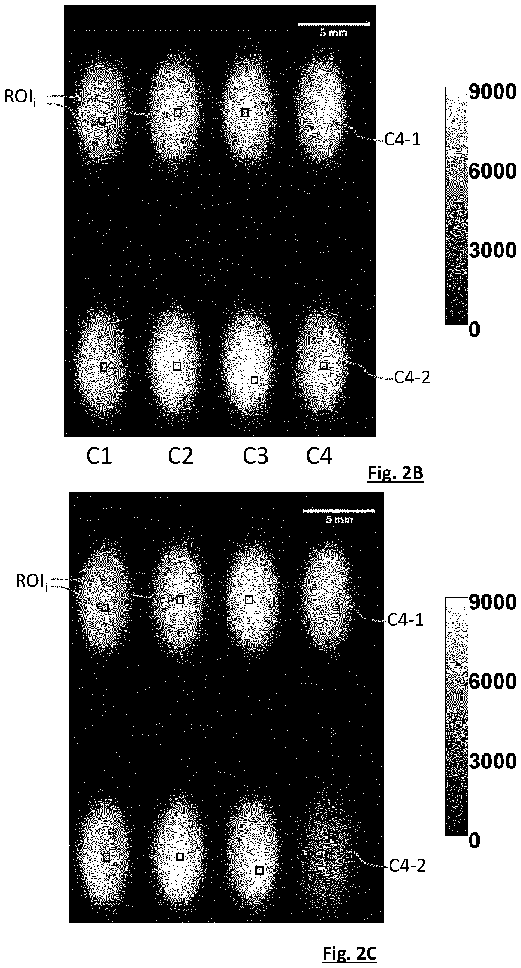

Les figures 2B et 2C illustrent un exemple de mise en œuvre du premier mode de réalisation. Les composants et paramètres expérimentaux sont les suivants :

- carte fluidique 22 : carte usinée dans une plaque d'aluminium, comportant huit chambres circulaires de diamètre 3 mm (soit une section de 7.1 $mm^2$) ;
- volume de chaque chambre fluidique : 35 $\mu$L;
- capteur d'image : capteur RGB CMOS Canon 1200D au format APS-C dimensions 14.9 mm x 22.3 mm - 18 millions de pixels;
- source de lumière 10 : Ecran LCD (Liquid Cristal Display) associé avec un filtre passe-bande (Thorlabs FB 560-10) centré sur une longueur d'onde de 560 nm et définissant une largeur à mi-hauteur de 10 nm ;
- température : 25 °C ;
- distance source de lumière - échantillon : 1 cm ;
- épaisseur de chaque chambre fluidique : 5 mm.

Chaque échantillon a été prélevé dans 9 mL de milieu nutritif Bouillon Tryptone Soja (usuellement désigné par l'acronyme TSB - Trypcase Soy Broth), dans lequel on avait introduit 250 $\mu$L d'une solution de culture de *Pseudomonas putida* ATCC12633, et 100 $\mu$L d'une suspension de phages *Pseudomonas virus gh1.* Une chambre de contrôle n'a pas fait l'objet d'ajout de phages.

Une chambre de référence a été remplie avec le milieu nutritif liquide, sans bactériophages ni bactéries. Les suspensions de bactériophages avaient respectivement un titre infectieux égal à $6.10^9$ ufp/mL, $6.10^8$ ufp /mL et $6.10^7$ ufp /mL. L'unité ufp est une unité connue de l'homme du métier signifiant unité formant plage, et usuellement désigné plaque forming unit. La détermination du titre de chaque suspension de phages a été effectuée selon une méthode de référence, en utilisant un milieu gélosé. Chaque chambre a fait l'objet d'un duplicat, sauf la chambre de contrôle et la chambre de référence.

Le dispositif a été placé dans une enceinte thermostatée à 25°C. Des images ont été acquises toutes les 2 minutes durant une durée de 6 heures et 4 minutes. Les figures 2B et 2C représentent des images respectivement acquises à t = 30 minutes (image initiale) et t = 6h et 4 minutes. L'instant initial t = 0 correspond à l'inoculation des bactéries dans le milieu liquide. Les images ont été formées en ne prenant en compte que les pixels verts du capteur. Sur les figures 2B et 2C, on distingue différentes régions d'intérêt $ROI_i$, chaque région d'intérêt correspondant à un échantillon élémentaire $20_i$. Dans cet exemple, les régions d'intérêt des colonnes C1, C2 et C3 correspondent à un échantillon élémentaire identique : même souche bactérienne - même concentration de phages.

Sur les figures 2B et 2C, les puits des colonnes C1, C2 et C3 correspondent respectivement aux concentrations de $6.10^7$ ufp/mL (soit MOI = 0.01) , $6.10^8$ ufp /mL (soit MOI = 0.1) et $6.10^9$ ufp /mL (soit MOI = 1). L'acronyme MOI signifie Multiplicity of Infection, terme connu de l'homme du métier désignant un rapport entre une quantité de phages (en ufp /mL) sur une quantité de bactéries (en ufc/mL) dans chaque échantillon. Le terme ufc signifie unité formant colonie, (ou colony forming unit en anglais), usuellement utilisé en microbiologie.

La quatrième colonne C4 comporte la chambre de référence C4-1 et la chambre de contrôle C4-2 ne comportant pas de phage.

Sur la chambre de contrôle C4-2, on constate un assombrissement de l'image. Cela est dû à la prolifération des bactéries en l'absence de phage.

Sur chaque colonne, on a effectué une moyenne de l'intensité (c'est-à-dire des niveaux de gris) des images acquises toutes les 2 minutes. On a calculé ensuite une densité optique équivalente DO(t) , cette dernière étant obtenue par l'expression :

$$DO(t) = -log_{10} \frac{I(t)}{I(t=0)} + 0.055$$

Où $I(t)$ est l'intensité à un instant de mesure $t$, correspondant à une moyenne de niveaux de gris et $I(t = 0)$ est l'intensité à l'instant initial $t = 0$. La constante 0.055 a été ajoutée afin que la densité optique à l'instant initial corresponde à celle mesurée par un spectrophotomètre.

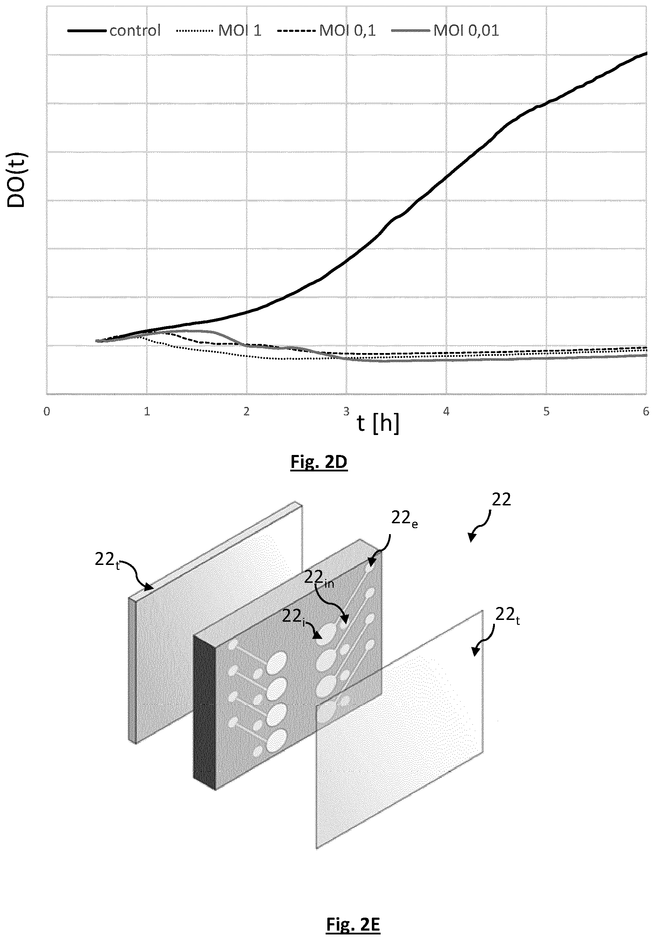

La figure 2D montre l'évolution de la densité optique équivalente DO(t) (axe des ordonnées) en fonction du temps (axe des abscisses - unité : heure).

A partir de ces courbes, on constate que sous l'action du phage, et pour l'ensemble des concentrations de phage considérées, la densité optique croît durant quelques heures après l'instant initial, mais se stabilise ensuite. Dans la chambre de contrôle, la densité optique augmente de façon continue. La comparaison des densités optiques respectivement mesurées dans la chambre de contrôle et dans les autres chambres montre l'inhibition du développement des bactéries par le phage. L'action du phage devient perceptible deux heures après l'instant initial.

On constate que l'invention permet un suivi en temps réel de l'action d'un phage sur des bactéries, et cela simultanément sur plusieurs échantillons élémentaires $20_i$. Il est possible de définir, pour chaque échantillon, un seuil de détection, dont le franchissement signifie qu'une prolifération bactérienne est suspectée dans l'échantillon. Un tel seuil peut être défini à partir de la densité optique moyenne mesurée sur l'échantillon de contrôle, ainsi que d'un indicateur de dispersion $\sigma$, par exemple l'écart type, à un instant de mesure ou en différents instants de mesure. Le seuil de détection peut être égal à $\mu + k\sigma$, k étant un réel strictement positif, de préférence supérieur à 1 ou 2, et par exemple égal à 5. L'établissement d'un tel seuil de détection est aisément automatisable et permet de déterminer précocement la présence d'une prolifération bactérienne, par exemple entre 5h et 10h. D'autres seuils, basés sur une comparaison entre l'échantillon de contrôle et des échantillons comportant des phages peuvent être définis.

La figure 2E schématise une carte fluidique 22 permettant une mise en œuvre de l'invention. La carte fluidique comporte des chambres fluidiques $22_i$, telles que précédemment décrites, respectivement fermées par deux parois transversales $22_t$. Une paroi transversale peut prendre la forme d'un film adhésif transparent. L'autre paroi transversale $22_t$ peut être réalisée en utilisant un matériau transparent, de préférence perméable à l'oxygène, de façon à permettre une oxygénation des chambres fluidique $22_i$, nécessaire au développement de certaines bactéries. Ainsi, il est préférable qu'au moins une paroi transversale délimitant chaque chambre fluidique soit perméable à l'oxygène. Il peut par exemple s'agir de PDMS (Polydiméthylsiloxane) ou de TPX (Polyméthylpentène), ce dernier étant décrit dans le document EP0745667.

Le milieu nutritif et/ou les phages peuvent être préalablement disposés dans chaque chambre fluidique $22_i$ en étant par exemple à l'état lyophilisé. Dans l'exemple représenté sur la figure 2E, chaque chambre fluidique $22_i$ est reliée, par un canal fluidique, à une entrée $22_i$, et évent $22_e$. Le remplissage de chaque chambre fluidique est effectué à la seringue, en introduisant la solution comportant les bactéries par l'entrée $22_{in}$. L'évent $22_e$ permet de chasser l'air lors du remplissage de la chambre fluidique. Un filtre bloquant les liquides et perméable aux gaz peut être disposé au niveau de l'évent $22_e$.

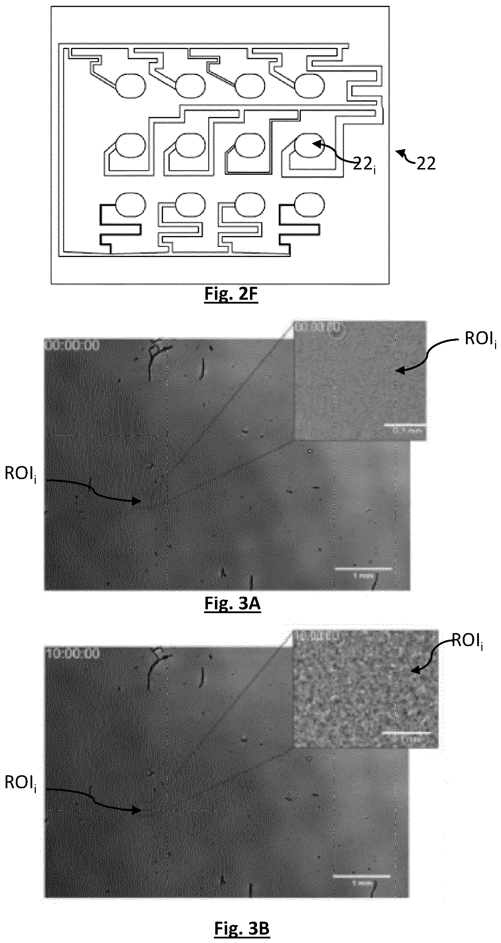

De façon alternative, comme représenté sur la figure 2F, chaque chambre fluidique est initialement sous vide. Le remplissage de chaque chambre fluidique est assuré par une entrée commune. Lorsque la carte fluidique 22 est placée sous vide, la solution aqueuse comportant les bactéries migre vers chaque chambre fluidique. Les bactériophages sont préalablement disposés dans chaque chambre fluidique, à l'état lyophilisé. La mise en contact entre les bactéries et les bactériophages a donc lieu au cours du remplissage de chaque chambre fluidique.

Selon une autre possibilité, différentes chambres fluidiques comportent respectivement différentes souches bactériennes. Ces dernières peuvent être présentes à un état lyophilisé. Dans ce cas, le remplissage de chaque chambre fluidique peut être assuré par une entrée commune, à travers laquelle s'écoule une solution comportant des phages. L'objectif est alors d'identifier des souches virales pouvant être pertinentes pour certaines souches bactériennes. Selon cette possibilité, une étape préalable d'enrichissement de phages peut être prévue. Au cours de cette étape, les différentes souches bactériennes sont disposées dans une même enceinte, dans laquelle une solution de phages, comportant une seule souche virale ou plusieurs souches virales, est mélangée. Si, parmi les souches bactériennes présentes, une souche est sensible à un phage, ce dernier est répliqué, ce qui entraîne une augmentation de sa concentration. On obtient alors une amplification non spécifique de phages. Le mélange est ensuite filtré, de manière à retenir les bactéries, la taille de filtration pouvant être de l'ordre de 0.2 $\mu$m. Les souches bactériennes sont ensuite réparties dans différentes chambres fluidiques, de manière à aboutir à une seule souche bactérienne par puits. La solution filtrée est injectée dans chaque chambre fluidique, de manière à identifier la souche bactérienne sensible au phage.

Ce mode de réalisation peut être adapté pour tester l'effet de différentes souches virales sur une même souche bactérienne. Pour cela, différentes chambres fluidiques peuvent respectivement comporter des phages de différentes souches de phages. Au sein d'une chambre fluidique comportant une souche bactérienne sensible au phage, aucune prolifération bactérienne n'est observée, et le phage est amplifié de façon spécifique. Lorsque la souche bactérienne d'une chambre n'est pas sensible au phage, on observe une prolifération bactérienne.

Dans ce mode de réalisation, on mesure une densité optique ou, de façon plus générale, une évolution d'une intensité lumineuse transmise par chaque échantillon élémentaire $20_i$ et détectée par le capteur d'image. Afin d'obtenir une illumination uniforme de chaque échantillon élémentaire $20_i$, c'est-à-dire de chaque chambre fluidique élémentaire $22_i$, il est préférable que la source de lumière 10 soit une source étendue, par exemple un écran à cristaux liquides. Cela favorise la compacité du dispositif. De façon alternative, une source de lumière 10 ponctuelle peut être utilisée, sous réserve de l'éloigner suffisamment de l'échantillon.

Les figures 3A et 3B illustrent une variante du premier mode de réalisation, dans lequel chaque échantillon élémentaire est présent, à l'état liquide, dans une chambre fluidique. Selon cette variante, la présence de microorganismes est quantifiée par une analyse de la texture de l'image. Sous l'effet du développement des bactéries, il a été observé que la texturation des images est plus marquée. Sur chaque région d'intérêt $ROI_i$ d'une image acquise, un indicateur de texturation est établi, de façon à caractériser l'échantillon élémentaire $20_i$ correspondant à la région d'intérêt. L'indicateur de texturation peut être un vecteur ou une grandeur scalaire. Ce mode de réalisation est principalement destiné à des échantillons de faible épaisseur, par exemple comprise entre 100 $\mu$m et 1 mm, et de préférence entre 100 $\mu$m et 250 $\mu$m. Sur les figures 3A et 3B, les régions d'intérêt considérées ont été agrandies.

La texturation de l'image résulte de la formation de figures d'interférences sur le capteur d'image, entre l'onde lumineuse 12 transmise par l'échantillon, et l'onde lumineuse 13 résultant de la diffusion de l'onde lumineuse incidente 11 par l'échantillon. Selon ce mode de réalisation, il est préférable que la source de lumière soit spatialement cohérente (ponctuelle), et suffisamment éloignée du détecteur pour que l'onde lumineuse incidente 11 atteigne l'échantillon sous la forme d'ondes planes, ou considérées comme telles.

Les figures 3A à 3C illustrent un exemple de mise en œuvre de la variante du premier mode de réalisation. Les composants et paramètres expérimentaux sont :

- carte fluidique 22 : lamelle de microscope sur laquelle une chambre fluidique Geneframe d'épaisseur 250 $\mu$m est déposée, la chambre fluidique étant refermée par une autre lamelle de microscope ;
- volume de chaque chambre fluidique : 25 $\mu$L ;
- capteur d'image : capteur monochrome 10.5 Mpixels de 1.67 $\mu$m de côté, formant une surface de détection de 29.5 $mm^2$ (6.4 mm x 4.6 mm) ;
- source de lumière 10 : diode électroluminescente centrée sur la longueur d'onde de 650 nm avec une largeur à mi-hauteur de 40 nm ;
- température : 30 °C ;
- distance source de lumière - échantillon : 5 cm;
- épaisseur de chaque chambre fluidique : 250 $\mu$m.

Dans cet exemple, la chambre fluidique n'est pas compartimentée. Il n'y a qu'un seul échantillon analysé. L'échantillon a été prélevé dans 9 mL de milieu nutritif Bouillon Tryptone Soja (usuellement désigné par l'acronyme TSB - Trypcase Soy Broth), dans lequel on avait introduit 250 $\mu$L d'une solution de culture de *Pseudomonas putida* ATCC12633, diluée d'un facteur

10$^2$. On a ajouté 100 μL d'une suspension de phages *Pseudomonas virus gh1,* connu pour infecter les bactéries *Pseudomonas putida,* dont la concentration était estimée à 7 10$^5$ ufp/mL. 30 μL d'échantillon ont été prélevés et introduits dans la carte fluidique 22.

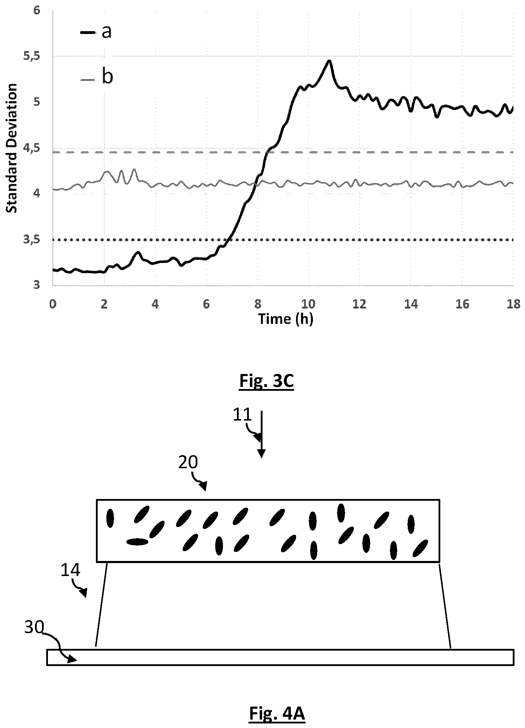

Les figures 3A et 3B montrent une image de la chambre fluidique respectivement à un instant initial ainsi que 10 heures après l'instant initial. Ces images ont été obtenues en mettant en œuvre un échantillon témoin, sans phage. Elles illustrent l'évolution de la texturation de l'image lorsque les bactéries se développent. Pour chaque image, un descripteur de texture a été déterminé en déterminant un écart type de trois zones réduites de 80 pixels x 80 pixels de l'image. Sur les figures 3A et 3B, on a représenté une telle zone réduite. Le descripteur de texture correspond à une moyenne des trois écarts-types ainsi déterminés. La figure 3C montre une évolution de l'indicateur de texturation déterminé :

- pour un échantillon témoin, sans phage (courbe a)
- pour un échantillon avec des phages *Pseudomonas virus gh1* - 7 10$^5$ ufp/mL (courbe b)

Cette figure a été obtenue en effectuant une acquisition d'images par période de 10 minutes durant une durée de 18 heures. On observe que :

- pour l'échantillon témoin, le descripteur de texture augmente, sous l'effet de la prolifération bactérienne ;
- pour l'échantillon comportant les phages, le descripteur de texture stagne au cours du temps, traduisant une inhibition du développement des bactéries.

Il est possible de définir un seuil de détection, dont le franchissement signifie qu'une prolifération bactérienne est suspectée dans l'échantillon. Un tel seuil est déterminé à partir d'une valeur moyenne (ou médiane) μ et d'un indicateur de dispersion, par exemple l'écart type σ, du descripteur de texture, durant une courte durée temporelle après l'instant initial, par exemple 1h ou 2 h. Le seuil de détection peut être égal à μ + kσ, k étant un réel strictement positif, de préférence supérieur à 1 ou 2, et par exemple égal à 5. L'établissement d'un tel seuil de détection est aisément automatisable et permet de déterminer précocement la présence d'une prolifération bactérienne, par exemple entre 5h et 10h.

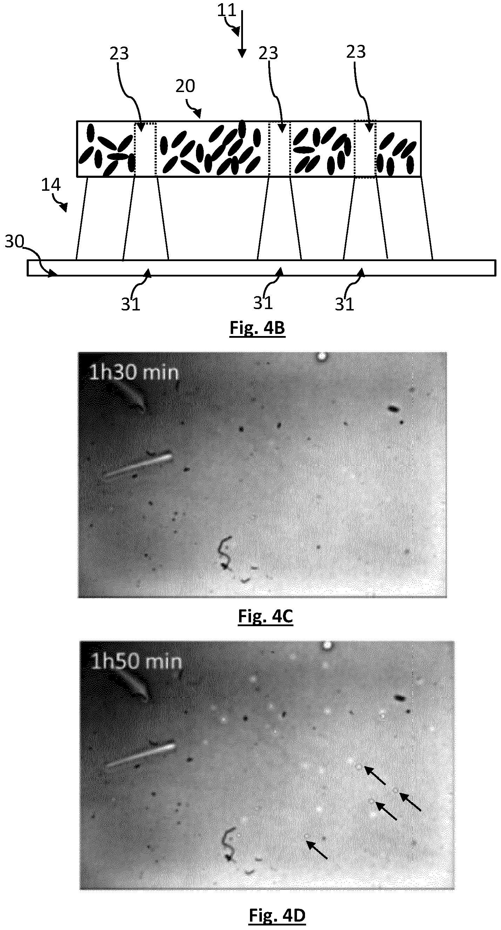

Les figures 4A à 4E illustrent un deuxième mode de réalisation, dans lequel les bactéries et les phages sont disposés dans une gélose, comportant un milieu nutritif. Le milieu nutritif est transparent et est de préférence non spécifique. La gélose comporte des phages, à l'état de virions. Ces derniers infectent les bactéries et se multiplient suite à la lyse de ces dernières. La lyse de bactéries se propage ainsi localement, au voisinage des localisations de virions à l'état initial. La propagation permet une formation d'une plage de lyse, correspondant à une partie de la gélose appauvrie en bactéries. De ce fait, au niveau des plages de lyse, la transmission de la lumière est augmentée par rapport aux autres parties de l'échantillon, dans lesquelles les bactéries prolifèrent.

Une telle gélose peut être constituée en mélangeant un milieu nutritif gélosé classique, porté en dessus de sa température de surfusion, par exemple entre 40°C et 60°C, avec une solution aqueuse comportant des bactéries et des phages. Ainsi, l'échantillon comporte une gélose molle, comprenant des bactéries et des phages. L'épaisseur de l'échantillon peut être comprise entre 2 mm à 5 mm. Le fait de disposer de bactéries et de phages selon une telle profondeur permet une meilleure mise en évidence de la lyse des bactéries. La fraction massique d'agar-agar est par exemple comprise entre 0.5% et 1.4%, ce qui est usuellement désigné par le terme "agar mou" (soft agar).

Selon une variante, une partie de l'épaisseur de l'échantillon est formée d'un milieu nutritif standard, sans bactérie ni phage. Il peut par exemple comprendre de l'agar-agar selon une fraction massique de 1.5 %. La fonction de ce milieu nutritif est de former un réservoir de nutriments pour les bactéries. Cependant, du fait que le procédé d'analyse d'un échantillon est rapide, de l'ordre de quelques heures ou de quelques dizaines d'heures, la présence d'un tel réservoir de nutriments n'est pas nécessaire. L'absence d'un tel réservoir est considérée comme avantageuse, car cela évite une diffusion, par ce dernier, de la lumière.

Les figures 4A à 4B schématisent un échantillon 20 respectivement à un instant initial, considéré comme correspondant à la formation de la couche superficielle 20s, et à un instant postérieur à l'instant initial. Au voisinage des phages initialement présents, des bactéries sont lysées, et la lyse des bactéries se propage de proche en proche, sous l'effet de la réplication des phages. Des zones appauvries 23, ou plages de lyse, se forment dans la couche superficielle 20s de l'échantillon. La transmission de la lumière à travers les plages de lyse est supérieure qu'à travers le reste de l'échantillon. Ainsi, lorsque l'échantillon est illuminé par une source de lumière, des groupes de pixels 31, situés face aux zones appauvries 23, collectent une quantité de lumière supérieure à la quantité de lumière captée par les autres pixels. Sur l'image acquise par le capteur d'image, cela se traduit par l'apparition de régions d'intérêt claires. Chaque région d'intérêt claire correspond à une plage de lyse. Le comptage des régions d'intérêt claires permet une estimation d'un titre infectieux de la solution comportant les bactériophages. Ce titre infectieux peut être exprimé en nombre d'unités formant plages (ufp).

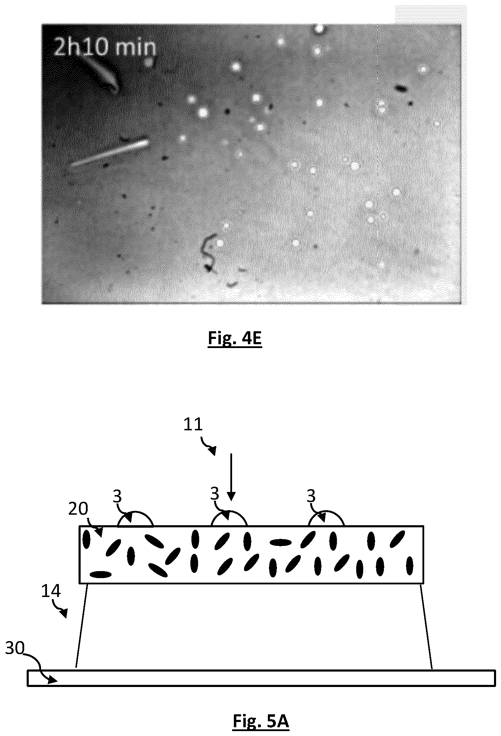

Les figures 4C, 4D et 4E sont des images expérimentales acquises respectivement 1h30, 1h50 et 2h10 après la préparation d'un échantillon. On a mélangé, dans 5 ml de soft agar (50% TSA - Trypticase Soy Agar - 50% TSB Trypticase Soy Broth) : 250 μl d'une culture de bactéries *Pseudomonas putida* ATCC12633 (environ 10$^9$ ufc/mL) en bouillon, ainsi que 100 μl d'une solution aqueuse comportant des phages *Pseudomonas*

*virus gh1.* La préparation a été homogénéisée par formation d'un vortex. 2mL de la préparation a été versée dans une boîte de Pétri de diamètre 3.5 mm, formant une chambre fluidique. L'échantillon a été disposé sur un capteur d'image 30 Mpixels et l'ensemble a été placé dans une enceinte thermostatée à 30 °C. Les paramètres expérimentaux sont les suivants :

- capteur d'image : capteur RGB CMOS Canon 1200D de dimensions 14.9 mm x 22.3 mm - 18 millions de pixels;
- source de lumière 10 : diode électroluminescente centrée sur la longueur d'onde de 560 nm;
- distance source de lumière - échantillon : environ 20 cm ;
- épaisseur de chaque chambre fluidique : 2 mm.

L'instant initial t = 0 est considéré comme pouvant être confondu avec le dépôt de la préparation dans la boîte de Pétri. La répartition de la biomasse dans la boîte est considérée comme homogène. Au cours du temps, des plages de lyse se forment, ce qui correspond à des taches claires sur les images 4C, 4D et 4E. Sur la figure 4D, certaines taches claires sont indiquées par des flèches. Ces images ont été formées à partir des pixels verts du capteur d'image. On observe la formation progressive de régions d'intérêt claires sur les images.

Selon une autre approche, on utilise une carte fluidique compartimentée, telle que décrite en lien avec le premier mode de réalisation. La carte fluidique comporte des chambres fluidiques $22_i$ séparées les unes des autres. Chaque chambre fluidique dispose d'un mélange de phages et d'un gélifiant à froid lyophilisé. Chaque chambre fluidique peut être alimentée par une solution comportant des bactéries. Il se forme alors une gélose, comportant un mélange de bactéries et de phages. Le gélifiant à froid est hydrosoluble. Il est configuré pour gélifier lorsqu'il est mis au contact d'une solution aqueuse, à température ambiante, formant alors un hydrogel. Il peut s'agir d'un polysaccharide hydrosoluble. Le gélifiant à froid peut être choisi parmi le carboxyméthylcellulose, la gomme de guar, la gomme arabique, la gomme gellane, la gomme de xanthane, ou un gélifiant obtenu à partir d'os d'animaux, par exemple porc, bœuf ou poulet.

Une telle approche permet de diviser l'échantillon en différentes zones spatiales, chaque zone spatiale correspondant à une chambre fluidique.

De façon alternative, la carte fluidique est portée, lors de son remplissage, à une température de surfusion de la gélose comportant les bactéries. Ainsi, la gélose comportant les bactéries peut remplir chaque chambre fluidique, de façon à se mélanger, dans chaque chambre, aux phages préalablement disposés à l'état lyophilisé. Cela évite le recours à un gélifiant à froid, ce dernier pouvant être diffusant.

Un troisième mode de réalisation est représenté sur les figures 5A à 5D. Selon ce mode de réalisation, les bactéries sont initialement présentes dans une gélose, ou dans une couche superficielle d'une gélose solidifiée. On dépose, à la surface de cette dernière, des gouttes d'une solution comportant des phages 3. Les gouttes sont séparées les unes des autres. Lorsque les bactéries contenues dans la gélose sont sensibles vis-à-vis du bactériophage, des plages de lyse se forment dans l'échantillon, comme décrit en lien avec le deuxième mode de réalisation. Un avantage de ce mode de réalisation est que la position de chaque goutte déposée peut être connue, ce qui permet de rechercher le développement de plages de lyses face à chacune de ces positions. Cela facilite l'étape de traitement d'image.

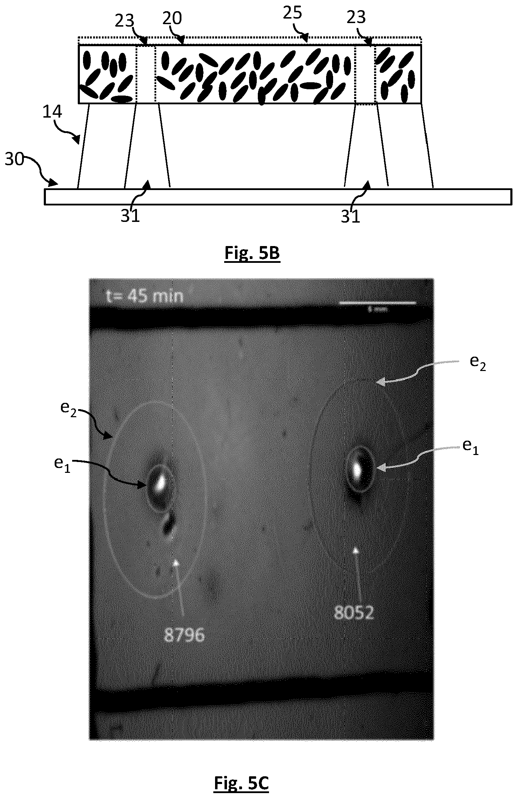

Les figures 5A et 5B schématisent ce mode de réalisation, respectivement lors du dépôt des gouttes sur la gélose, et suite à la formation de plages de lyse lorsque les bactéries sont sensibles à la souche virale des phages présents dans les gouttes. Les gouttes déposées peuvent comporter respectivement différentes souches virales, et/ou différentes concentrations de phages d'une même souche virale. De même que le premier mode de réalisation, ce mode de réalisation permet d'effectuer, en parallèle, des tests de différents phages ou de différentes concentrations. Sur la figure 5B, on a schématisé une formation de deux plages de lyse. De même que dans le deuxième mode de réalisation, sous l'effet de la lyse des bactéries, les plages de lyse apparaissent, à travers laquelle la transmission de la lumière est plus élevée que dans le reste de l'échantillon. Les pixels 31 situés face aux plages de lyse collectent un signal dont l'intensité est plus élevée. Il en résulte l'apparition de taches claires sur l'image formée par le capteur d'image.

On a préparé un échantillon en mélangeant 120 μL d'un milieu de culture, comportant *Pseudomonas putida* ATCC12633 et 6mL d'agar-agar mou (7.5 g/L d'agar-agar). Cette préparation a été coulée dans une boîte de pétri, ce qui a permis d'obtenir une gélose d'épaisseur 2 mm environ. On a ensuite déposé, à la surface, des gouttes de 5 μL comportant :

- soit une suspension de phages *Pseudomonas virus gh1* dont le titre était estimé à 3.6 $10^6$ ufp/mL ;
- soit un diluant pharmaceutique pour simuler la présence d'un phage non actif sur la souche bactérienne utilisée.

Les gouttes ont été séchées (15 minutes), puis l'échantillon a été déposé dans une enceinte thermostatée à 28 °C.. Les paramètres expérimentaux sont les suivants :

- capteur d'image : capteur RGB Canon CMOS 1200D de dimensions 14.9 mm x 22.3 mm - 18 millions de pixels;
- source de lumière 10 : diode électroluminescente centrée sur la longueur d'onde de 560 nm;
- distance source de lumière - échantillon : environ 20 cm ;

- épaisseur de la chambre fluidique : 2 mm.

Une fine épaisseur (entre 0.1 mm et 1 mm) d'huile 25 peut être disposée sur l'échantillon, de façon à éviter un dessèchement. L'huile 25 peut être de l'huile de silicone, permettant une diffusion de l'oxygène jusqu'au milieu nutritif.

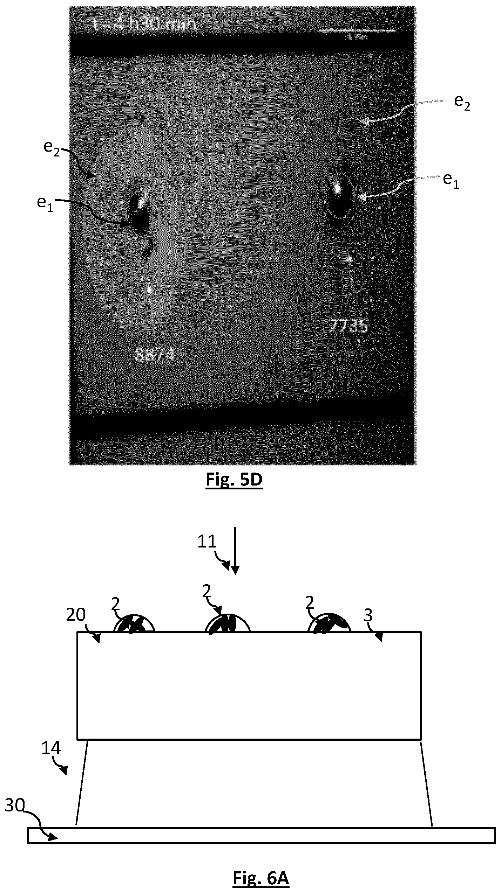

Les figures 5C et 5D sont des images expérimentales acquises respectivement 45 minutes et 4h30 après le dépôt de gouttes sur un échantillon. Sur chacune de ces figures, chaque ellipse e1 représente la zone de dépôt de la goutte et chaque ellipse e2 correspond à une région d'analyse de l'image. Sur chacune de ces images, les ellipses e1 matérialisées à gauche et à droite correspondent respectivement au dépôt du phage et de la solution de diluant pharmaceutique. En comparant les figures 5C et 5D, on observe une augmentation du niveau de gris au niveau de l'ellipse e2 à gauche de l'image. Cela correspond à la formation d'une plage de lyse. En revanche, au niveau de l'ellipse e2 située à droite de l'image, le niveau de gris diminue, suite au développement des bactéries.

Une tel mode de réalisation permet de diviser l'échantillon en différentes zones spatiales, chaque zone spatiale correspondant à une position au niveau de laquelle une goutte a été déposée. L'image acquise par le capteur d'image permet d'analyser simultanément plusieurs zones spatiales.

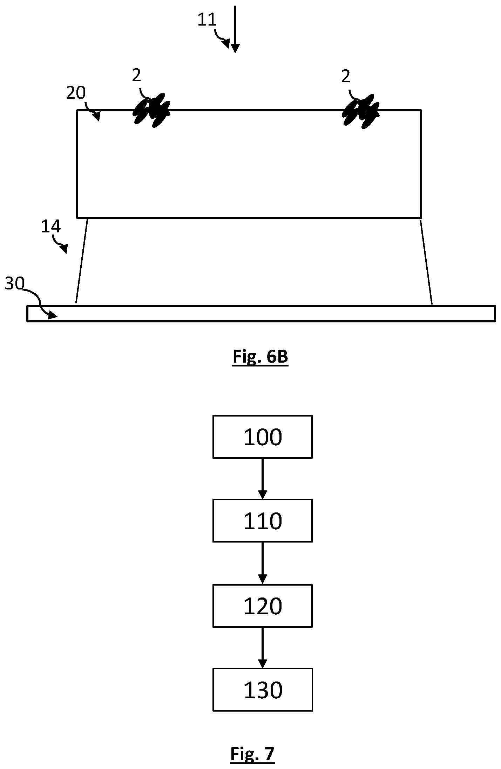

Un quatrième mode de réalisation est schématisé sur les figures 6A et 6B. Selon ce mode de réalisation, on dispose d'une gélose comportant des phages 3. On dépose, à la surface de cette dernière, des gouttes d'une solution comportant des bactéries. Les gouttes sont séparées les unes des autres. Lorsque les bactéries ne sont pas sensibles à la souche virale, elles se développent à la surface de l'échantillon, en formant des colonies. Lorsque les bactéries contenues dans les gouttes sont sensibles à la souche virale des bactériophages présents dans la gélose, on n'observe pas de formation de colonies à la surface de l'échantillon. Un avantage de ce mode de réalisation est que la position et la constitution de chaque goutte déposée peut être connue, ce qui permet de rechercher le développement de colonies à chacune de ces positions. Cela facilite l'étape de traitement d'image.

Les figures 6A et 6B schématisent ce mode de réalisation, respectivement lors du dépôt des gouttes sur la gélose, et suite à la formation de colonies lorsque les bactéries présentes dans une goutte sont peu ou pas sensibles au phage présent dans la gélose. Les gouttes déposées peuvent comporter respectivement différentes souches de bactéries, ou différentes concentrations d'une même souche. Ce mode de réalisation permet d'effectuer, en parallèle, des tests de différentes souches bactériennes ou de différentes concentrations d'une même souche bactérienne. Sur la figure 6B, on a schématisé une formation de deux colonies. Les pixels 31 situés face aux colonies collectent un signal dont l'intensité est plus faible. Il en résulte l'apparition de taches sombres sur l'image formée par le capteur d'image.

Une tel mode de réalisation permet de diviser l'échantillon en différentes zones spatiales, chaque zone spatiale correspondant à une localisation au niveau de laquelle une goutte a été déposée. L'image acquise par le capteur d'image permet d'analyser simultanément plusieurs zones spatiales.

La figure 7 schématise les principales étapes d'un procédé selon les modes de réalisation précédent.

Etape 100 : mise en contact de souches bactériennes avec des phages. Cette étape peut être réalisée dans une seule chambre fluidique (cf. deuxième, troisième, ou quatrième modes de réalisation), ou dans différentes chambres fluidiques (cf. premier mode de réalisation). Au cours de cette étape, l'échantillon peut être divisé en différentes zones spatiales. Ces zones spatiales peuvent correspondre :

- à des chambres fluidiques différentes les unes des autres (cf. premier mode de réalisation);
- ou à des positions différentes au niveau desquelles des gouttes d'une solution aqueuse de phages sont déposées sur une gélose comportant des bactéries (troisième mode de réalisation) ;
- ou à des positions différentes au niveau desquelles des gouttes d'une solution aqueuse de bactéries sont déposées sur une gélose comportant des phages (quatrième mode de réalisation)

Etape 110 : illumination de l'échantillon à l'aide de la source de lumière ;

Etape 120 : acquisition d'une image par le capteur d'image ;

Etape 130 : analyse de l'image par l'unité de traitement, de façon à évaluer une sensibilité d'au moins une souche bactérienne à au moins une souche virale.

Les avantages du procédé tel que précédemment décrit sont :

- une détection précoce de la lyse de bactéries par des phages. En effet, en utilisant les procédés de l'art antérieur, on considère que des résultats exploitables sont obtenus en au moins une journée
- une possibilité de mise en oeuvre de façon à adresser simultanément différentes souches bactériennes en combinaison avec différentes souches de phages, ou différents ratios entre les quantités de souches de phage et la quantité de souches de bactéries ;
- simplicité de mise en œuvre, sans nécessiter de matériel onéreux.

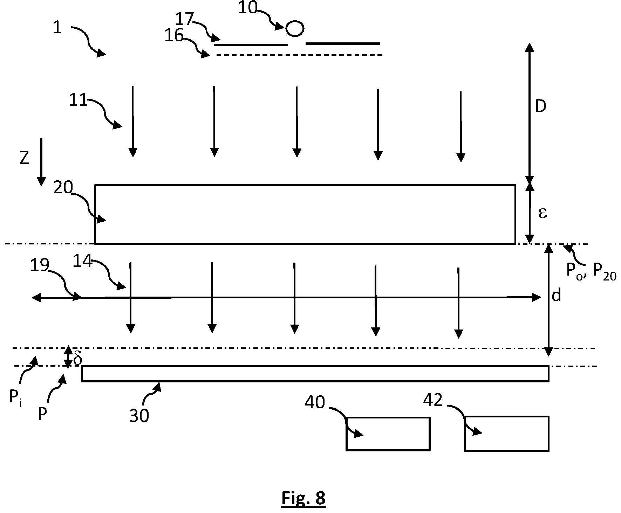

La figure 8 montre un autre dispositif permettant une mise en œuvre de l'invention. Le dispositif comporte un système optique 19 disposé entre l'échantillon 20 et le capteur d'image 30. Le système optique peut être une lentille ou un objectif. Le capteur d'image définit un plan de détection P. L'échantillon définit un plan d'échantillon $P_{20}$. Le plan de l'échantillon est un plan, de préférence parallèle au plan de détection, et s'étendant dans l'échantillon. Il s'agit de préférence de la surface de l'échantillon la plus proche du capteur d'image. Le système optique définit un plan image $P_i$ et un plan objet $P_o$. Selon une configuration défocalisée :

- le plan image est décalé par rapport au plan de détection d'une distance de défocalisation image;
- et/ou le plan objet est décalé par rapport au plan de l'échantillon d'une distance de défocalisation objet.

La distance de défocalisation image et/ou la distance de défocalisation objet est de préférence inférieure à 1 mm, voire inférieure à 500 $\mu$m.
Sur la figure 8, on a représenté une configuration défocalisée, dans laquelle le plan objet $P_o$ est confondu avec le plan de l'échantillon $P_{20}$. Le plan image est décalé par rapport au plan de détection d'une distance de défocalisation image $\delta$. D'une façon générale, la configuration défocalisée est préférée lorsque l'on cherche à quantifier une texturation de l'image.
Par rapport aux configurations exposées en lien avec la figure 8, la configuration sans lentille, décrite sur la figure 1, est préfére car elle permet d'utiliser un dispositif compact et peu onéreux.

**Revendications**

1. Procédé de détermination de la sensibilité d'une souche bactérienne à l'égard d'une souche virale de bactériophages (3), le procédé comportant :

    a) préparation d'un échantillon (20), comportant une mise en contact de bactéries (2), de la souche bactérienne, avec des bactériophages (3), chaque bactériophage appartenant à une même souche virale, les bactéries étant soit dans un milieu liquide, soit dans un milieu gélosé ;
    b) disposition de l'échantillon entre une source de lumière (10) et un capteur d'image (30), la source de lumière émettant une onde lumineuse (11) dans une bande spectrale d'émission comprise entre 500 nm et 600 nm, le capteur d'image s'étendant selon un plan de détection ;
    c) illumination de l'échantillon à l'aide de la source de lumière et acquisition d'au moins une image de l'échantillon, par le capteur d'image, dans la bande spectrale d'émission,, le capteur d'image étant :

    - soit placé selon une configuration d'imagerie sans lentille, aucune optique de grossissement ou de formation d'image n'étant disposé entre l'échantillon et le capteur d'image ;
    - soit placé selon une configuration d'imagerie défocalisée, un système optique, lentille ou objectif, étant disposé entre l'échantillon et le capteur d'image, le système optique définissant un plan objet et un plan image, le plan objet étant décalé par rapport au plan de détection ou le plan image étant décalé par rapport à l'échantillon ;

    d) à partir de chaque image acquise, détermination d'une sensibilité de la souche bactérienne à l'égard de la souche virale des bactériophages,

le procédé étant tel que :

    - lors de l'étape a), les bactéries et les bactériophages sont mélangés dans une solution aqueuse ;
    - l'étape c) comporte une acquisition d'au moins deux images, à des instants de mesure successifs ;
    - l'étape d) comporte une détermination d'une intensité lumineuse détectée par tout ou partie du capteur d'image, sur chaque image acquise respectivement à chaque instant de mesure (t), de telle sorte que la souche bactérienne est considérée comme :

        • peu ou pas sensible à la souche virale des bactériophages lorsque l'intensité lumineuse détectée diminue entre deux instants de mesure successifs;
        • ou sensible à la souche virale des bactériophages lorsque l'intensité lumineuse détectée ne diminue pas ou augmente entre deux instants de mesure successifs.

2. Procédé selon la revendication 1, dans lequel la largeur de la bande spectrale d'émission est inférieure à 50 nm.

3. Procédé selon l'une quelconque des revendications précédentes, dans lequel la distance entre l'échantillon (20) et le capteur d'image (30) est inférieure à 5 cm ou inférieure à 1 cm.

4. Procédé selon l'une quelconque des revendications précédentes, dans lequel l'étape d) comporte une détermination d'une atténuation de la lumière, émise par la source de lumière, par l'échantillon, de telle sorte que la souche bactérienne (2) est considérée comme :

• peu ou pas sensible à la souche virale des bactériophages lorsque l'atténuation augmente entre deux instants de mesure successifs ;

• ou sensible à la souche virale des bactériophages lorsque l'atténuation n'augmente pas entre deux instants de mesure successifs.

5. Procédé selon l'une quelconque des revendications précédentes, dans lequel :

- lors de l'étape a), les bactéries et les bactériophages sont mélangés dans une solution aqueuse ;

- l'étape c) comporte une acquisition d'au moins deux images, à des instants de mesure successifs ;

- l'étape d) comporte une détermination d'un descripteur de texturation de chaque image acquise, de telle sorte que la souche bactérienne est considérée comme :

• pas ou peu sensible à la souche virale des bactériophages lorsque l'évolution du descripteur de texturation traduit une augmentation d'une diffusion de la lumière par l'échantillon entre deux instants de mesure successifs ;

• ou sensible à la souche virale des bactériophages lorsque l'évolution du descripteur de texturation traduit une diminution ou une stagnation d'une diffusion de la lumière par l'échantillon entre deux instants de mesure successifs.

6. Procédé selon l'une quelconque des revendications 1 à 4, dans lequel

- lors de l'étape a), les bactéries et les bactériophages sont mélangés dans une gélose ;

- l'étape c) comporte une acquisition d'au moins une image, postérieurement à l'étape a) ;

- l'étape d), comporte une analyse de chaque image acquise, de façon à identifier des régions d'intérêt claires, chaque région d'intérêt claire correspondant à une infection de bactéries par des bactériophages, formant une plage de lyse, chaque région d'intérêt claire indiquant une sensibilité de la souche bactérienne à l'égard de la souche virale des bactériophages.

7. Procédé selon la revendication 6, comportant :

- un comptage du nombre de régions d'intérêt claires sur au moins une image acquise ;

- une estimation d'un titre infectieux des bactériophages dans l'échantillon à partir du nombre de régions d'intérêt claires comptées.

8. Procédé selon l'une quelconque des revendications 1 à 4, dans lequel :

- lors de l'étape a), les bactéries sont disposées dans une gélose, et les bactériophages sont disposés dans une solution, l'étape a) comportant un dépôt d'au moins une goutte de la solution sur la gélose ;

- l'étape c) comporte une acquisition d'au moins une image, postérieurement à l'étape a) ;

- l'étape d) comporte une analyse de chaque image acquise, de façon à identifier des régions d'intérêt claires, chaque région d'intérêt claire correspondant à une infection de bactéries par des bactériophages, formant une plage de lyse, de telle sorte que l'apparition, de chaque région d'intérêt claire indique une sensibilité de la souche bactérienne à l'égard de la souche virale des bactériophages.

9. Procédé selon la revendication 8, dans lequel l'étape a) comporte un dépôt de plusieurs gouttes, les gouttes étant espacées les unes des autres, deux gouttes différentes comportant respectivement :

- des bactériophages de différentes souches virales ;

- et/ou différentes concentrations de bactériophages d'une même souche virale.

10. Procédé selon l'une quelconque des revendications 1 à 4, dans lequel :

- lors de l'étape a), les bactériophages sont disposés dans une gélose, et les bactéries sont disposées dans une solution, l'étape a) comportant un dépôt d'une goutte de la solution sur la gélose;

- l'étape c) comporte une acquisition d'au moins une image, postérieurement à l'étape a) ;

- l'étape d) comporte une analyse de chaque image acquise, de façon à identifier:

• des régions d'intérêt claires, chaque région d'intérêt claire correspondant à une infection de bactéries par des bactériophages, de telle sorte que chaque région d'intérêt claire indique une sensibilité de la souche bactérienne à l'égard de la souche virale des bactériophages;

• ou des régions d'intérêt sombres, chaque région d'intérêt sombre correspondant à un développement de bactéries en présence de bactériophages, de telle sorte que chaque région d'intérêt sombre indique une insensibilité ou une faible sensibilité de la souche bactérienne d'intérêt à l'égard de la souche virale des bactériophages.

11. Procédé selon la revendication 10, dans lequel l'étape a) comporte un dépôt de plusieurs gouttes, les gouttes étant espacées les unes des autres, deux gouttes différentes comportant respectivement :

- des bactéries de différentes souches bactériennes
- et/ou différentes concentrations de bactéries d'une même souche bactérienne.

12. Procédé selon l'une quelconque des revendications précédentes, dans lequel l'échantillon est divisé en différentes zones spatiales ($20_i$), séparées les unes des autres de telle sorte que:

- au moins deux zones spatiales différentes comportent respectivement une même souche bactérienne et une concentration différente de bactériophages d'une même souche virale;
- et/ou au moins deux zones spatiales différentes comportent respectivement une même souche bactérienne et des bactériophages de différentes souches virales ;
- et/ou au moins deux zones spatiales différentes comportent respectivement des bactériophages d'une même souche virale et différentes souches bactériennes ;
le procédé étant tel que chaque zone spatiale est associée à une région d'intérêt ($ROI_i$) de chaque image acquise, deux zones spatiales différentes étant associées à deux régions d'intérêt différentes, de façon que l'analyse d'une même image acquise permet d'obtenir une information relative à la sensibilité d'une souche bactérienne à l'égard d'une souche virale de bactériophages, et cela dans différentes zones spatiales.

13. Procédé selon la revendication 12, dans lequel l'échantillon comporte plus de 10, voire plus de 100, zones spatiales différentes, séparées les unes des autres, chaque zone spatiale étant paramétrée trois paramètres correspondant respectivement à la souche bactérienne d'intérêt, à la souche virale des bactériophages et à la concentration des bactériophages, au moins un paramètre de deux zones spatiales différentes étant différent.

14. Procédé selon l'une quelconque des revendications 12 ou 13, dans lequel l'échantillon est réparti dans différentes chambres fluidiques ($22_i$), d'une carte fluidique, chaque chambre fluidique correspondant à une zone spatiale de l'échantillon.

15. Procédé selon l'une quelconque des revendications 12 ou 13, dans lequel l'échantillon est formé suite à un dépôt de gouttes à la surface d'une gélose, en différentes positions, les gouttes étant espacées les unes des autres, de telle sorte qu'une zone spatiale est définie par chaque position de gouttes.

16. Procédé selon l'une quelconque des revendications précédentes comportant, préalablement à l'étape a), une étape d'enrichissement, comportant :

- un mélange de bactériophages de même souche virale, ou de différentes souches virales, dans une solution aqueuse comportant au moins une souche de bactéries ;
- une incubation ;
- une filtration du mélange, de façon à retenir les bactéries et obtenir une solution enrichie en bactériophages ;
de telle sorte que lors de l'étape a), la mise en contact des bactériophages avec les bactéries est effectuée en utilisant la solution enrichie en bactériophages.

**Patentansprüche**

1. Verfahren zur Bestimmung der Empfindlichkeit eines Bakterienstamms gegenüber einem Bakteriophagen-Virusstamm (3), wobei das Verfahren Folgendes umfasst:

a) Herstellung einer Probe (20), umfassend das Inkontaktbringen von Bakterien (2) des Bakterienstamms mit Bakteriophagen (3), wobei jeder Bakteriophage demselben Virusstamm angehört, wobei die Bakterien sich entweder in einem flüssigen Medium oder in einem Agarmedium befinden,
b) Einbringen der Probe zwischen eine Lichtquelle (10) und einen Bildsensor (30), wobei die Lichtquelle eine Lichtwelle (11) in einem Emissionsspektralbereich zwischen 500 nm und 600 nm emittiert, wobei der Bildsensor sich entlang einer Nachweisebene erstreckt,
c) Belichten der Probe mithilfe der Lichtquelle und Aufnahme mindestens eines Bildes der Probe durch den Bildsensor im Emissionsspektralbereich, wobei der Bildsensor:

- gemäß einer Bildgebungskonfiguration ohne Linse platziert ist, wobei keine Vergrößerungs- oder Bildaufnahmeoptik zwischen der Probe und dem Bildsensor angeordnet ist,
- gemäß einer defokussierten Bildgebungskonfiguration platziert ist, wobei ein optisches System, eine Linse oder ein Objektiv, zwischen der Probe und dem Bildsensor angeordnet ist, wobei das optische System eine Objektebene und eine Bildebene definiert, wobei die Objektebene in Bezug auf die Nachweisebene versetzt ist oder die Bildebene in Bezug auf die Probe versetzt ist,

d) Bestimmung, ausgehend von jedem aufgenommenen Bild, einer Empfindlichkeit des Bakterienstamms gegenüber dem Bakteriophagen-Virusstamm,

wobei das Verfahren dergestalt ist, dass:

- in Schritt a) die Bakterien und die Bakteriophagen in einer wässrigen Lösung gemischt werden,
- Schritt c) die Aufnahme von mindestens zwei Bildern zu aufeinanderfolgenden Messzeitpunkten umfasst,
- Schritt d) die Bestimmung einer von dem gesamten Bildsensor oder einem Teil davon auf jedem zu dem jeweiligen Messzeitpunkt ($t$) aufgenommenen Bild nachgewiesenen Lichtintensität derart umfasst, dass der Bakterienstamm als:

  • wenig oder gar nicht empfindlich gegenüber dem Bakteriophagen-Virusstamm betrachtet wird, wenn die nachgewiesene Lichtintensität zwischen zwei aufeinanderfolgenden Messzeitpunkten abnimmt,
  • oder als empfindlich gegenüber dem Bakteriophagen-Virusstamm betrachtet wird, wenn die nachgewiesene Lichtintensität zwischen zwei aufeinanderfolgenden Messzeitpunkten nicht abnimmt oder ansteigt.

2. Verfahren nach Anspruch 1, wobei die Breite des Emissionsspektralbereichs kleiner als 50 nm ist.

3. Verfahren nach einem der vorhergehenden Ansprüche, wobei der Abstand zwischen der Probe (20) und dem Bildsensor (30) kleiner als 5 cm oder kleiner als 1 cm ist.

4. Verfahren nach einem der vorhergehenden Ansprüche, wobei Schritt d) die Bestimmung einer Abschwächung des von der Lichtquelle ausgestrahlten Lichts durch die Probe derart umfasst, dass der Bakterienstamm (2) als:

  • wenig oder gar nicht empfindlich gegenüber dem Bakteriophagen-Virusstamm angesehen wird, wenn die Abschwächung zwischen zwei aufeinanderfolgenden Messzeitpunkten zunimmt,

• oder als empfindlich gegenüber dem Bakteriophagen-Virusstamm angesehen wird, wenn die Abschwächung zwischen zwei aufeinanderfolgenden Messzeitpunkten nicht zunimmt.

5. Verfahren nach einem der vorhergehenden Ansprüche, wobei:

- in Schritt a) die Bakterien und die Bakteriophagen in einer wässrigen Lösung gemischt werden,
- Schritt c) die Aufnahme von mindestens zwei Bildern zu aufeinanderfolgenden Messzeitpunkten umfasst,
- Schritt d) die Bestimmung eines Deskriptors der Texturierung jedes aufgenommenen Bildes derart umfasst, dass der Bakterienstamm als:

  • gar nicht oder wenig empfindlich gegenüber dem Bakteriophagen-Virusstamm angesehen wird, wenn die Veränderung des Texturierungsdeskriptors eine Zunahme der Lichtstreuung durch die Probe zwischen zwei aufeinanderfolgenden Messzeitpunkten widerspiegelt,
  • oder als empfindlich gegenüber dem Bakteriophagen-Virusstamm angesehen wird, wenn die Veränderung des Texturierungsdeskriptors eine Abnahme oder eine Stagnation der Lichtstreuung durch die Probe zwischen zwei aufeinanderfolgenden Messzeitpunkten widerspiegelt.

6. Verfahren nach einem der Ansprüche 1 bis 4, wobei

- in Schritt a) die Bakterien und die Bakteriophagen in einem Agar gemischt werden,
- Schritt c) die Aufnahme mindestens eines Bildes nach Schritt a) umfasst,
- Schritt d) eine Analyse jedes aufgenommenen Bildes umfasst, um klare Regionen von Interesse zu identifizieren, wobei jede klare Region von Interesse einer Infektion von Bakterien durch die Bakteriophagen entspricht, die ein Lyseplaque bildet, wobei jede klare Region von Interesse auf Empfindlichkeit des Bakterienstamms gegenüber dem Bakteriophagen-Virusstamm hindeutet.

7. Verfahren nach Anspruch 6, das Folgendes umfasst:

- Zählen der Anzahl der klaren Regionen von Interesse auf mindestens einem aufgenommenen Bild,
- Schätzung des infektiösen Titers der Bakteriophagen in der Probe anhand der Anzahl der gezählten klaren Regionen von Interesse.

**8.** Verfahren nach einem der Ansprüche 1 bis 4, wobei:

- in Schritt a) die Bakterien in einen Agar eingebracht werden und die Bakteriophagen in eine Lösung eingebracht werden, wobei Schritt a) das Aufbringen mindestens eines Tropfens der Lösung auf den Agar umfasst,
- Schritt c) die Aufnahme mindestens eines Bildes nach Schritt a) umfasst,
- Schritt d) eine Analyse jedes aufgenommenen Bildes umfasst, um klare Regionen von Interesse zu identifizieren, wobei jede klare Region von Interesse einer Infektion von Bakterien durch die Bakteriophagen entspricht, die ein Lyseplaque bildet, derart, dass das Auftreten jeder klaren Region von Interesse auf Empfindlichkeit des Bakterienstamms gegenüber dem Bakteriophagen-Virusstamm hindeutet.

**9.** Verfahren nach Anspruch 8, wobei Schritt a) das Aufbringen mehrerer Tropfen umfasst, wobei die Tropfen voneinander beabstandet sind, wobei zwei verschiedene Tropfen jeweils:

- Bakteriophagen verschiedener Virusstämme
- und/oder verschiedene Bakteriophagen-Konzentrationen desselben Virusstamms umfassen.

**10.** Verfahren nach einem der Ansprüche 1 bis 4, wobei:

- in Schritt a) die Bakteriophagen in einen Agar eingebracht werden und die Bakterien in eine Lösung eingebracht werden, wobei Schritt a) das Aufbringen eines Tropfens der Lösung auf den Agar umfasst,
- Schritt c) die Aufnahme mindestens eines Bildes nach Schritt a) umfasst,
- Schritt d) eine Analyse jedes aufgenommenen Bildes umfasst, um Folgendes zu identifizieren:

• klare Regionen von Interesse, wobei jede klare Region von Interesse einer Infektion von Bakterien mit Bakteriophagen entspricht, so dass jede klare Region von Interesse auf Empfindlichkeit des Bakterienstamms gegenüber dem Bakteriophagen-Virusstamm hindeutet,
• oder dunkle Regionen von Interesse, wobei jede dunkle Region von Interesse einem Bakterienwachstum in Gegenwart von Bakteriophagen entspricht, so dass jede dunkle Region von Interesse auf Unempfindlichkeit oder geringe Empfindlichkeit des Bakterienstamms von Interesse gegenüber dem Bakteriophagen-Virusstamm hindeutet.

**11.** Verfahren nach Anspruch 10, wobei Schritt a) das Aufbringen mehrerer Tropfen umfasst, wobei die Tropfen voneinander beabstandet sind, wobei zwei verschiedene Tropfen jeweils:

- Bakterien verschiedener Bakterienstämme
- und/oder verschiedene Konzentrationen von Bakterien desselben Bakterienstamms umfassen.

**12.** Verfahren nach einem der vorhergehenden Ansprüche, wobei die Probe in verschiedene räumliche Zonen (20i) unterteilt ist, die voneinander getrennt sind, derart, dass:

- mindestens zwei verschiedene räumliche Zonen jeweils denselben Bakterienstamm und eine andere Konzentration von Bakteriophagen desselben Virusstamms umfassen
- und/oder mindestens zwei verschiedene räumliche Zonen jeweils denselben Bakterienstamm und Bakteriophagen verschiedener Virusstämme umfassen
- und/oder mindestens zwei verschiedene räumliche Zonen jeweils Bakteriophagen desselben Virusstamms und verschiedene Bakterienstämme umfassen,

wobei das Verfahren derart ist, dass jede räumliche Zone mit einer Region von Interesse ($ROI_l$) jedes aufgenommenen Bildes assoziiert ist, wobei zwei verschiedene räumliche Zonen mit zwei verschiedenen Regionen von Interessen assoziiert sind, so dass es die Analyse desselben aufgenommenen Bildes gestattet, eine Information hinsichtlich der Empfindlichkeit eines Bakterienstamms gegenüber einem Bakteriophagen-Virusstamm und dies in verschiedenen räumlichen Zonen zu erhalten.

**13.** Verfahren nach Anspruch 12, wobei die Probe mehr als 10 oder sogar mehr als 100 voneinander getrennte, verschiedene räumliche Zonen umfasst, wobei jede räumliche Zone durch drei Parameter parametriert wird, die dem Bakterienstamm von Interesse, dem Bakteriophagen-Virusstamm bzw. der Konzentration der Bakteriophagen entsprechen, wobei mindestens ein Parameter zweier verschiedener räumlicher Zonen unterschiedlich ist.

**14.** Verfahren nach einem der Ansprüche 12 oder 13, wobei die Probe in verschiedene Fluidkammern ($22_l$) eines Fluidchips verteilt wird, wobei jede Fluidkammer einer räumlichen Zone der Probe entspricht.

**15.** Verfahren nach einem der Ansprüche 12 oder 13, wobei die Probe nach dem Aufbringen von Tropfen auf der Oberfläche eines Agars an verschiedenen Positionen gebildet wird, wobei die Tropfen voneinander beabstandet sind, so dass durch jede Tropfenposition eine räumliche Zone definiert wird.

**16.** Verfahren nach einem der vorhergehenden Ansprüche, umfassend vor Schritt a) einen Anreicherungsschritt, der Folgendes umfasst:

- ein Gemisch von Bakteriophagen desselben Virusstamms oder verschiedener Virusstämme in einer wässrigen Lösung, die mindestens einen Bakterienstamm umfasst,
- eine Inkubation,
- eine Filtration des Gemischs, um die Bakterien zurückzuhalten und eine an Bakteriophagen angereicherte Lösung zu erhalten,

so dass in Schritt a) das Inkontaktbringen der Bakteriophagen mit den Bakterien unter Verwendung der an Bakteriophagen angereicherten Lösung durchgeführt wird.

## Claims

**1.** Method for determining the sensitivity of a bacterial strain to a viral strain of bacteriophages (3), the method comprising:

a) preparing a sample (20), this comprising bringing bacteria (2), of the bacterial strain, into contact with bacteriophages (3), each bacteriophage belonging to the same viral strain, the bacteria being either in a liquid medium, or in an agar medium;
b) placing the sample between a light source (10) and an image sensor (30), the light source emitting a light wave (11) in an emission spectral band comprised between 500 nm and 600 nm, the image sensor lying in a detection plane;
c) illuminating the sample using the light source and acquiring at least one image of the sample, with the image sensor, in the emission spectral band, the image sensor being:

- either arranged in a lensless imaging configuration, no magnifying or image-forming optics being placed between the sample and the image sensor;
- or arranged in a defocused imaging configuration, an optical system, i.e. a lens or objective, being placed between the sample and the image sensor, the optical system defining an object plane and an image plane, the object plane being offset from the detection plane or the image plane being offset from the sample;

d) on the basis of each acquired image, determining a sensitivity of the bacterial strain to the viral strain of the bacteriophages,

the method being such that:

- in step a), the bacteria and the bacteriophages are mixed in an aqueous solution;
- step c) comprises acquiring at least two images, at successive measurement times;
- step d) comprises determining a light intensity detected by all or part of the image sensor, in each image respectively acquired at each measurement time (t), such that the bacterial strain is considered to be:

• hardly or not sensitive to the viral strain of the bacteriophages when the detected light intensity decreases between two successive measurement times;
• or sensitive to the viral strain of the bacteriophages when the detected light intensity does not decrease or increases between two successive measurement times.

**2.** Method according to Claim 1, wherein the width of the emission spectral band is narrower than 50 nm.

**3.** Method according to either of the preceding claims, wherein the distance between the sample (20) and the image sensor (30) is smaller than 5 cm or smaller than 1 cm.

**4.** Method according to any of the preceding claims, wherein step d) comprises determining an attenuation of the light, emitted by the light source, by the sample, such that the bacterial strain (2) is considered to be:

• hardly or not sensitive to the viral strain of the bacteriophages when the attenuation increases between two successive measurement times;
• or sensitive to the viral strain of the bacteriophages when the attenuation does not increase between two successive measurement times.

**5.** Method according to any of the preceding claims, wherein:

- in step a), the bacteria and the bacteriophages are mixed in an aqueous solution;
- step c) comprises acquiring at least two images, at successive measurement times;
- step d) comprises determining a texture descriptor for each acquired image, such that the bacterial strain is considered to be:

• hardly or not sensitive to the viral strain of the bacteriophages when the variation in the texture descriptor is indicative of an increase in scattering of the light by the sample between two successive measurement times;
• or sensitive to the viral strain of the bacteriophages when the variation in the texture descriptor is indicative of a decrease or a stagnation in scattering of the light by the sample between two successive measurement times.

6. Method according to any of Claims 1 to 4, wherein:

   - in step a), the bacteria and the bacteriophages are mixed in an agar medium;
   - step c) comprises acquiring at least one image, subsequently to step a);
   - step d) comprises analysing each acquired image, so as to identify light regions of interest, each light region of interest corresponding to an infection of bacteria by bacteriophages, forming a viral plaque, each light region of interest indicating a sensitivity of the bacterial strain to the viral strain of the bacteriophages.

7. Method according to Claim 6, comprising:

   - counting the number of light regions of interest in at least one acquired image;
   - estimating a viral load of the bacteriophages in the sample on the basis of the number of light regions of interest counted.

8. Method according to any of Claims 1 to 4, wherein:

   - in step a), the bacteria are located in an agar medium, and the bacteriophages are located in a solution, step a) comprising depositing at least one droplet of the solution on the agar medium;
   - step c) comprises acquiring at least one image, subsequently to step a);
   - step d) comprises analysing each acquired image, so as to identify light regions of interest, each light region of interest corresponding to an infection of bacteria by bacteriophages, forming a viral plaque, such that the appearance of each light region of interest indicates a sensitivity of the bacterial strain to the viral strain of the bacteriophages.

9. Method according to Claim 8, wherein step a) comprises depositing a plurality of droplets, the droplets being spaced apart from one another, two different droplets respectively comprising:

   - bacteriophages of various viral strains;
   - and/or various concentrations of bacteriophages of a given viral strain.

10. Method according to any of Claims 1 to 4, wherein:

    - in step a), the bacteriophages are located in an agar medium, and the bacteria are located in a solution, step a) comprising depositing a droplet of the solution on the agar medium;
    - step c) comprises acquiring at least one image, subsequently to step a);
    - step d) comprises analysing each acquired image, so as to identify:

      • light regions of interest, each light region of interest corresponding to an infection of bacteria by bacteriophages, such that each light region of interest indicates a sensitivity of the bacterial strain to the viral strain of the bacteriophages;
      • or dark regions of interest, each dark region of interest corresponding to a development of bacteria in the presence of bacteriophages, such that each dark region of interest indicates an insensitivity or a low sensitivity of the bacterial strain of interest to the viral strain of the bacteriophages.

11. Method according to Claim 10, wherein step a) comprises depositing a plurality of droplets, the droplets being spaced apart from one another, two different droplets respectively comprising:

    - bacteria of various bacterial strains;
    - and/or various concentrations of bacteria of a given bacterial strain.

12. Method according to any of the preceding claims, wherein the sample is divided into various spatial regions (20i) that are separated from one another such that:

    - at least two different spatial regions respectively comprise the same bacterial strain and a different concentration of bacteriophages of a given viral strain;
    - and/or at least two different spatial regions respectively comprise the same bacterial strain and bacteriophages of various viral strains;
    - and/or at least two different spatial regions respectively comprise bacteriophages of the same viral strain and various bacterial strains;

    the method being such that each spatial region is associated with one region of interest (ROI$_i$) of each acquired image, two different spatial regions being associated with two different regions of interest, so that analysis of a given acquired image allows information relating to the sensitivity of a bacterial strain to a viral strain of bacteriophages to be obtained in various spatial regions.

**EP 3 822 360 B1**

13. Method according to Claim 12, wherein the sample comprises more than 10, or even more than 100, different spatial regions that are separated from one another, each spatial region being parameterized by three parameters respectively corresponding to the bacterial strain of interest, to the viral strain of the bacteriophages and to the concentration of the bacteriophages, at least one parameter of two different spatial regions being different.

14. Method according to either of Claims 12 and 13, wherein the sample is distributed between various fluidic chambers ($22_i$) of a fluidic chip, each fluidic chamber corresponding to one spatial region of the sample.

15. Method according to either of Claims 12 and 13, wherein the sample is formed following a deposition of droplets on the surface of an agar medium, in various positions, the droplets being spaced apart from one another, such that one spatial region is defined by each droplet position.

16. Method according to any of the preceding claims, comprising, prior to step a), an enriching step, comprising:

   - mixing bacteriophages of a given viral strain, or of various viral strains, in an aqueous solution comprising at least one strain of bacteria;
   - incubating;
   - filtering the mixture, so as to retain the bacteria and obtain a solution enriched in bacteriophages;

such that, in step a), when the bacteriophages are brought into contact with the bacteria the solution enriched in bacteriophages is used.

**Fig. 1**

**Fig. 2A**

**Fig. 2B**

**Fig. 2C**

**Fig. 2D**

**Fig. 2E**

**Fig. 2F**

**Fig. 3A**

**Fig. 3B**

**Fig. 3C**

**Fig. 4A**

Fig. 4B

Fig. 4C

Fig. 4D

**Fig. 4E**

**Fig. 5A**

**Fig. 5B**

**Fig. 5C**

**Fig. 5D**

**Fig. 6A**

**Fig. 6B**

**Fig. 7**

**Fig. 8**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 20190188886 A
- EP 2747578 A
- WO 2013027146 A
- EP 0745667 A

**Littérature non-brevet citée dans la description**

- **HENRY M. et al.** *Development of a high throughput assay for indirectly measuring phage growth using the Omnilog TM system*
- **ESTRELLA LUIS et al.** *Characterization of novel Staphylococcus aureus lytic phage and defining their combinatorial virulence using the Omnilog system*